# EUROPEAN PATENT SPECIFICATION

(11) **EP 4 079 355 B1**
(45) Date of publication and mention of the grant of the patent: **29.05.2024**
(21) Application number: 21305533.8
(22) Date of filing: 23.04.2021
(51) Int. Cl.: A61F 7/00, A61M 16/00, A61F 7/12, A61M 16/04

(54) **TOTAL LIQUID VENTILATION SYSTEM AND METHOD**
SYSTEM UND VERFAHREN ZUR VOLLSTÄNDIGEN FLÜSSIGKEITSBELÜFTUNG
SYSTÈME ET PROCÉDÉ DE VENTILATION LIQUIDIENNE TOTALE

(43) Date of publication of application: 26.10.2022
(73) Proprietor: Orixha, 69450 Saint-Cyr-au-Mont-d'Or (FR)
(72) Inventor: PAUBLANT, Fabrice, 69450 Saint-Cyr-au-Mont-d'Or (FR); NADEAU, Mathieu, Compton, J0B 1L0 (CA); MARINE, Julien, 49100 Angers (FR); BAJOLET, Aymeric, 54600 Villers-Lès-Nancy (FR); MICHEAU, Philippe, Sherbrooke (Québec) J1N 2W5 (CA)
(74) Representative: Icosa

(56) References cited:
- WO-A2-2004/082729
- US-A- 6 149 624
- US-A1- 2012 226 337

## Description

### FIELD OF INVENTION

The present disclosure pertains to the field of total liquid ventilation. In particular, the invention relates to a method and system for providing heat exchange and gas exchange to a breathable liquid in a total liquid ventilation system.

### BACKGROUND OF INVENTION

Mechanical ventilators are machine used to support the ventilatory function of critically ill patients, to replace the function of the lungs until the patient's spontaneous ventilation can be restored.

Mechanical ventilators may be used to replace the lung function for a limited amount of time, but they face limitations due to their pro-inflammatory effect in some situations of respiratory distresses such as the ones induced by COVID and other infectious agents. Moreover, mechanical ventilators may cause lung damage, such as ventilator-induced lung injury (VILI).

Total Liquid Ventilation (TLV) strategies hold the promise of a radical shift in ventilation. In Total Liquid Ventilation, the mammal is ventilated with a breathable liquid having a high solubility for oxygen and carbon dioxide, such as perfluorocarbons, rather than with an oxygen-containing gas mixture.

Since the oxygen is completely dissolved in the breathable liquid, the air-liquid interface is removed, which results in an improved lung compliance. In addition to improving compliance, total liquid ventilation has been shown to not induce pulmonary inflammation. Moreover, with a view to other applications than respiratory support, TLV allows to use the lungs as heat exchangers with the blood pool.

These substantial differences between total liquid ventilators and conventional ventilators could, once liquid ventilators are proved to be safe in the clinic, enable their use in different medical settings: to induce ultra-rapid therapeutic hypothermia to protect critical organs (brain, heart, kidneys, liver...) from ischemia reperfusion damage from cardiac arrest; to provide respiratory support in acute respiratory distress syndrome or to enable lung lavage.

Total liquid ventilators traditionally comprise an oxygenator configured to remove carbon dioxide, and introduce dioxygen, in the breathable liquid. The breathable liquid may be cooled or heated simultaneously, or before, or after passing through the oxygenator.

The renewed breathable liquid, enriched in oxygen and brought at the desired temperature, can be reintroduced into the lungs of the subject via the endotracheal tube and an inspiratory pump and then extracted from the lungs through the endotracheal tube and an expiratory pump. Thus, it is possible to fill the lungs of the subject with the breathable liquid, which exchange heat and gas with the blood compartment of the subj ect.

Ice or cold-water baths, evaporators, or propane refrigerators may be used as cooling unit to cool a thermal fluid using metal-based heat transfer systems to exchange the heat between the breathable liquid and the thermal fluid cooled cooling unit and bring the breathable liquid at the desired temperature. These heat exchange systems are not compatible with the requirements of a medical device for regular use in the clinical setting. First, the temperature regulation is not precise and bears the risk of under or over cooling the patient. Secondly, these metal-based solutions are not compatible with a secure sterile approach to the treatment, *i.e.* they cannot guarantee that the breathable liquid that will be in contact with the lungs of the patient will not be contaminated by residues of a previous patient sticking to the walls of the metal heat exchangers. Another disadvantage of these systems is that the heat transfer with the breathable liquid is slow and inefficient.

The present invention aims at providing a solution to the problems that are currently limiting the use of total liquid ventilators.

In particular, an objective of the present invention is to provide a total liquid ventilator in which the heat exchange and gas exchange with the breathable liquid is optimized.

### SUMMARY

The invention is defined by the appended claims.

The present disclosure relates to a total liquid ventilation system configured to deliver a breathable liquid to the lungs of a mammal, the total liquid ventilation system comprising an inspiratory circuit, an expiratory circuit, and a breathable liquid treatment unit.

The breathable liquid treatment unit comprises:
- a gas injection system configured to inject a gas mixture into the breathable liquid, so as to obtain a two-phase mixture;
- a heat exchange system configured to heat and/or cool the two-phase mixture, the heat exchange system comprising:
   - a backing device configured to provide mechanical support;
   - at least one plate configured to be at a controlled temperature; and
   - a bag located in between the at least one plate and the backing device, the bag comprising at least two opposite surfaces, a fluid inlet fluidly connected to the expiratory circuit, and a fluid outlet fluidly connected to the inspiratory circuit;
- a pumping system comprising a pump for circulating the two-phase mixture from the fluid inlet to the fluid outlet through the fluid duct; and
the backing device, the at least one plate, and the bag being configured to define a fluid duct extending between the fluid inlet and the fluid outlet of the bag, the fluid duct having a length which is larger than the distance between the fluid inlet and the fluid outlet

Advantageously, the system is compatible with the requirements of a medical device for regular use in the clinical setting. Indeed, it ensures a secure and sterile approach. Moreover, the temperature can be controlled precisely.

In addition, the heat exchange system allows to provide a heat exchange and gas exchange with the breathable liquid, simultaneously.

Having a fluid duct which extends between the fluid inlet and the fluid outlet length for a length which is larger than the distance between said fluid inlet and fluid outlet, ensures that the residence time of the breathable liquid in the heat exchange system is long enough to complete the exchange of heat and gas.

In one embodiment, the system further comprises a recirculating circuit configured to connect the fluid outlet and the fluid inlet so as to define a closed circuit with the fluid duct; and the pump of the pumping system is for circulating the breathable liquid in the recirculating circuit.

Advantageously, the circulation of the breathable liquid inside the closed circuit formed by the fluid duct and the recirculating circuit allows to provide several successive treatments (for instance: carbon dioxide removal, oxygen addition, heat exchange or a combination thereof) to the breathable liquid, before introducing the breathable liquid in the inspiratory circuit.

The number of treatments per minute (corresponding to the number of loops in the closed circuit) depends on the flow rate at which the breathable liquid is pumped by the pump of the pumping system.

In one embodiment, the bag further comprises a gas outlet and a pressure control valve mounted on said gas outlet.

Advantageously, the gas outlet allows to remove CO₂ from the breathable liquid, before introducing the breathable liquid in the inspiratory circuit. Therefore, the inspiratory pump can pump in the endotracheal tube configured to be inserted in the mammal's trachea a breathable liquid with a low amount of CO₂.

For instance, the fluid duct may communicate with a reservoir configured to separate a gas from the breathable liquid, and the gas outlet may be located in proximity of said reservoir.

A capnometer may be installed at the gas outlet 133, so as to measure the concentration of CO2 in the evacuated gas.

In one embodiment, the bag comprises a sealed boundary, and wherein the fluid duct is defined by the non-sealed portion of the bag surrounded by said sealed boundary.

In this embodiment a pattern (the sealed boundary) is defined on the bag. More precisely, an inflatable compartment is defined within the non-sealed portion of the bag which is delimited by the sealed boundary. This inflatable compartment provides a fluid duct which can receive the breathable liquid.

Advantageously, the fluid duct thus obtained has an improved tightness, because it is delimited by a sealed boundary. The leaking of the breathable liquid out of the fluid duct is thus avoided.

In one embodiment, the at least one plate and/or the backing device comprises a relief pattern or a recess pattern.

The pattern on the plate at a controlled temperature and/or on the backing device may be in combination, or in alternative, to the pattern on the bag described hereabove.

For instance, the bag may comprise a sealed boundary, and the plate and/or on the backing device may comprise a relief pattern complementary to the sealed boundary on the bag. Alternatively, the bag does not comprise a sealed boundary and the fluid duct is defined inside the portion of the bag which is sandwiched between by the patterns on the plate and/or on the backing device.

In one embodiment, the backing device is a plate parallel to the at least one plate, and separated from said at least one plate by a distance comprised between 0 mm and 20 mm, preferably between 4 mm and 15 mm.

This distance allows to provide enough space for the bag and the fluid duct.

In one embodiment, the fluid duct comprises a serpentine duct extending between the fluid inlet and the fluid outlet and a fluid reservoir for separating a gas from the breathable liquid, said reservoir being fluidly connected with the serpentine duct and with the gas outlet of the bag.

In this case, the fluid duct has a length which is longer more than twice the distance between the fluid inlet and the fluid outlet. This fluid duct communicates with a reservoir configured to separate a gas from the breathable liquid, and the gas outlet allows to expel the separated gas.

In one embodiment, wherein the fluid reservoir has a volume comprised between 0.1 land 11.

This embodiment ensures the presence of a volume large enough to contain the separated gas, before it is expelled through the gas outlet.

In one embodiment, the system further comprises a second reservoir for buffering a breathable liquid from an expiratory pump.

Advantageously, the second reservoir allows to obtain a constant flow rate of the breathable liquid inside the fluid duct and in the gas exchange system, thereby improving the heat and gas exchange occurring in said fluid duct.

Moreover, the second reservoir prevents an overload of the expiratory pump.

More precisely, a fluid port allows to introduce the breathable liquid inside the second reservoir before reaching the fluid duct. The collection of the liquid inside the second reservoir, before introduction in the fluid duct, prevents an excessive power consumption by the expiratory pump.

In one embodiment, the system further comprises a third reservoir for buffering a breathable liquid to be delivered to an inspiratory pump.

Advantageously, the third reservoir advantageously allows to obtain a TLV system of compact dimensions, because the presence of buffer bags external to the heat exchange system is not required. More precisely, it is also possible to reduce the volume of breathable liquid circulating liquid inside the TLV system, and to provide a shorter inspiratory circuit.

Therefore, the thermal losses are minimized because the breathable liquid which has been treated in the breathable liquid treatment unit can reach more rapidly the inspiratory pump.

In one embodiment, the second reservoir and/or the third reservoir is integrated inside the bag.

This embodiment allows to obtain a "all-in-one" disposable bag. Advantageously, an all-in-one bag ensures that the breathable liquid is maintained at the same pressure during the breathable liquid treatment.

In one embodiment, the gas injection system comprises a gas mixing chamber fluidly connected to the fluid inlet of the bag.

The gas mixing chamber allows to create homogeneous gas mixtures.

In one embodiment, the gas mixing chamber comprises a nozzle configured to generate micro-bubbles.

This embodiment allows to obtain a two-phase mixture comprising the breathable liquid and the micro-bubbles.

In one embodiment, the bag is made of a plastic material selected from a group comprising: Thermoplastic polyurethane (TPU), Ethylene vinyl acetate (EVA), Polyvinyl Chloride (PVC).

Advantageously, these materials are resistant to heat and pressure. Moreover, they have a low thermal resistance.

In one embodiment, the volume of the disposable bag is comprised between 1 l and 7 l.

This volume ensure that enough breathable liquid can be contained inside the bag; more precisely, inside the fluid duct.

Furthermore, the bag may be a disposable bag. Advantageously, a disposable bag allows to minimize the manual operations, thereby reducing the risk of contaminations.

The present invention also relates to a method for providing thermal and gas exchange to a breathable liquid in a total liquid ventilation system according to any one of the embodiments described hereinabove, the method comprising:
- injecting, by means of the gas injection system, a gas mixture into the breathable liquid, so as to obtain a two-phase mixture;
- pumping, by means of the pump, the two-phase mixture in the fluid duct through the inlet of the bag.

Advantageously, this method allows to provide simultaneous heat and gas exchange to the breathable liquid. Indeed, it allows to introduce a gas mixture in a breathable liquid withdrawn by the expiratory pump so as to obtain a two-phase mixture. Once inside the fluid duct, the two-phase mixture can exchange heat with the plate at controlled temperature.

In one embodiment, the step of introducing a gas mixture into the breathing liquid comprises:
- introducing a gas mixture comprising oxygen into a mixing chamber comprising a nozzle;
- introducing the breathable liquid into the mixing chamber;
- generating a two-phase mixture comprising the breathable liquid and gas micro-bubbles by conducting the gas mixture and the breathable liquid through the nozzle of the mixing chamber.

Advantageously, this embodiment allows to oxygenate a breathable liquid withdrawn by the expiratory pump, by introducing a gas mixture comprising oxygen.

In one embodiment, the method further comprises:
- conducting the two-phase mixture from the fluid inlet to the fluid outlet of the bag;
- pumping, by means of the pump, the two-phase mixture from the fluid outlet to the fluid inlet.

Advantageously, this embodiment allows to circulate the breathable liquid inside a closed circuit comprising the fluid duct and the recirculating circuit. The breathable liquid can circulate a predetermined number of times inside said closed circuit, until it reaches the desired temperature and gas composition. Then, it can be introduced in the inspiratory circuit and delivered to the endotracheal tube via the inspiratory pump.

### DEFINITIONS

In the present invention, the following terms have the following meanings:
- **"Oxygenator"** refers to a device capable of providing oxygen and carbon dioxide gas exchange.
- **"gas-deprived"** herein refers to a fluid in which a gas has been removed via a gas removal process, so that the amount of said gas in the fluid (measured as a concentration, a partial pressure, ...) after gas removal is equal to or less than half of the initial gas amount, wherein the gas removal process may be a membrane-based process, a conversion, or any other known gas removal process.

### BRIEF DESCRIPTION OF THE DRAWINGS

**Figure 1** is a diagram showing a total liquid ventilation (TLV) system S of the invention according to one particular embodiment. The breathable liquid treatment unit 1 of the system S is represented inside the dotted line.
**Figure 2** is a diagram showing a TLS system S according to another embodiment.
**Figure 3** **is** an exploded view showing the heat exchange system 10 of the invention according to one embodiment.
**Figure 4** is a diagram showing a TLV system S according to one embodiment.
**Figures 5** is a simplified diagram showing a circulation of a breathable liquid inside a TLV system S. More precisely, **figure 5A** shows the circulation of the breathable liquid in the expiratory and inspiratory circuits 70, 60; **figure 5B** shows the circulation of the breathable liquid in the recirculating circuit in a first direction; and **figure 5C** shows the circulation of the breathable liquid in the recirculating circuit in a second direction.
**Figure 6A** is a three-dimensional representation of the bag 13 of the present invention according to one embodiment.
**Figure 6B** is a section representing the bag 13 of figure 6A on a vertical plane parallel to the xz plane.

### DETAILED DESCRIPTION

The following detailed description will be better understood when read in conjunction with the drawings. For the purpose of illustrating, the system is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

### TLV system

The present invention relates to a total liquid ventilation (TLV) system S configured to deliver a breathable liquid to the lungs of a mammal, said TLV system S comprising a breathable liquid treatment unit 1, an inspiratory circuit 60, an expiratory circuit 70, and a pump assembly 5.

Preferably, the breathable liquid comprises perfluorocarbons. Perfluorocarbons are particularly adapted for this purpose, as they are biocompatible, they have elevated oxygen and carbon dioxide solubility.

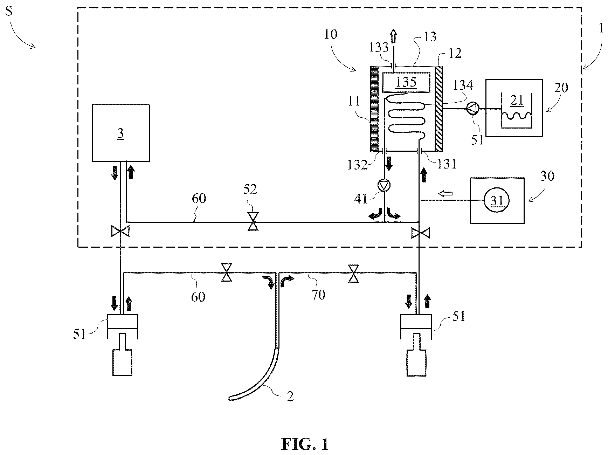

**Figure 1** shows a TLV system S according to the invention.

The breathable liquid treatment unit 1 allows to treat the breathable liquid circulating in the TLV system S. The treatment of the breathable liquid may comprise: removing carbon dioxide, adding oxygen, exchanging heat, or a combination thereof. The breathable liquid treatment unit 1 and its components will be later described.

The inspiratory and expiratory circuits 60, 70 of the TLV system S comprise conduits in which the breathable liquid circulates.

The pump assembly 5 comprises pumps 51 configured to move the breathable liquid through the conduits of the inspiratory and expiratory circuits 60, 70. More precisely, the pump assembly 5 comprises at least two pumps 51: an inspiratory pump 51 which is connected to the inspiratory circuit 60, and an expiratory pump 51 which is connected to the expiratory circuit 70. For instance, said inspiratory and expiratory pumps 51 may be piston pumps, as shown in **figure 1****.**

In **figure 1****,** an endotracheal tube 2, which is external to the TLV system S, is also illustrated. The endotracheal tube 2 is configured to be inserted in the trachea of the mammal.

Advantageously, the inspiratory and expiratory circuits 60, 70 of the TLV system S are configured to be connected to the endotracheal tube 2. For instance, they may be configured to be connected to the endotracheal tube 2 via a Y-connector.

The connection with the endotracheal tube 2 permits to put the inspiratory and expiratory circuits 60, 70 of the TLV system S in communication with the respiratory system of the mammal. Therefore, the breathable liquid may be delivered from the inspiratory circuit 60 to the mammal's lung, and from the mammal's lung to the expiratory circuit 70, via the aforementioned inspiratory and expiratory pumps 51, respectively.

More precisely, the flow of the breathable liquid in the TLV system S, when the system is connected to the endotracheal tube 2, comprises:
- circulating in the breathable liquid treatment unit 1, where the breathable liquid is treated;
- circulating in the inspiratory circuit 60 from the breathable liquid treatment unit 1 towards the endotracheal tube 2;
- being delivered, via the inspiratory pump 51, into the endotracheal tube 2, so as to fill the lungs of the mammal with the breathable liquid;
- being withdrawn, via the expiratory pump 51, from the endotracheal tube 2, so as to remove the breathable liquid from the lungs of the mammal.

Then, said withdrawn breathable liquid circulates in the expiratory circuit 70 until it reaches again the breathable liquid treatment unit 1 of the TLV system S. The flow of the breathable liquid described hereinabove allows to provide a ventilation cycle to the mammal.

Advantageously, the pump assembly 5 of the TLS system S further comprises a plurality of valves 52 configured to selectably allow the flow of the breathable liquid through predetermined portions of the inspiratory and expiratory circuits 60, 70.

For instance, the plurality of valves 52 may comprise a first valve located on the inspiratory circuit 60, between the inspiratory pump 51 and the endotracheal tube 2, and a second valve located on the expiratory circuit 70, between the endotracheal tube 2 and the expiratory pump 51. Accordingly, when one of said first or second valves 52 is closed, the inspiratory or expiratory circuit 60, 70 is isolated from the respiratory system of the mammal.

Other valves 52 may be located upstream and/or downstream of the components of the TLV system S, so as to selectably allow the flow of the breathable liquid through each component. For instance, one or more valves 52 may regulate the flow of the breathable liquid through the aforementioned breathable liquid treatment unit 1.

The TLV system S may further comprise a plurality of sensors configured to measure physical variables of the breathable liquid circulating inside the TLV system S. For instance, the TLV system S may comprise temperature sensors, pressure sensors, flow meters, capnometers, oxygen sensors, and the like.

The TLV system S may further comprise a controller. Preferably, the controller is configured to receive the signals measured by the sensors, and to process said signal. For instance, the controller may compare the signal to a set of reference values. Then, the controller may control the breathable liquid treatment unit 1 and/or the pump assembly 5 of the TLV system S based on the result of said comparison.

The control unit may further be configured to control the opening and the closing of the valves 52, so as to direct the flow of the breathable liquid in the fluid duct within selected compartments of the TLV system S.

The TLV system may further comprise a buffer bag 3 (also called inspiratory buffer bag 3) having a buffer bag inlet and a buffer bag outlet.

In the TLV system S of **figure 1****,** the buffer bag inlet is connected to an outlet of the breathable liquid treatment unit 1, and the buffer bag outlet is connected to an inspiratory pump 51. The buffer bag 3 is configured to receive, via the expiratory pump 51, an expired breathable liquid.

In this example, the bag 3 has the twofold function of a buffer bag, *i.e.,* it helps to create and maintain a buffer, and of a storage bag, *i.e.,* it is configured to contain and store a breathable liquid which fills the circuits of the TLV system S.

However, in an alternative embodiment, the bag 3 is not configured to store the breathable liquid which fills the circuits of the TLV system S. In this case, the TLV system S comprises a separate bag, container, or box, for breathable liquid storage purposes.

Preferably, the buffer bag 3 has a volume comprised between 1 et 5 1.

The breathable liquid treatment unit 1 will now be described in more details.

### The BLTU 1

The breathable liquid treatment unit 1 according to the invention comprises:
- a gas injection system 30, configured to inject a gas mixture, such as medical air and or medical oxygen, into the breathable liquid, so as to obtain a two-phase mixture;
- a heat exchange system 10, configured to thermally exchange with the two-phase mixture;
- and
- a pumping system 40, comprising a pump 41 for circulating the two-phase mixture from the fluid inlet 131 to the fluid outlet 132 through the fluid duct 134;
- optionally, a thermal unit 20, configured to control the amount of thermal exchange between the heat exchange system 10 and the breathable liquid.

### Heat exchange system 10

The heat exchange system 10 comprises at least one backing device 11 configured to provide mechanical support to the heat exchange system 10, and at least one plate 12 configured to be at a controlled temperature (i.e. a thermal plate). The heat exchange system 10 further comprises a bag 13 located in between the plate 12 and the backing device 11.

The bag 13 comprises a fluid inlet 131, configured to be fluidly connected to the expiratory circuit 70, and a fluid outlet 132 configured to be fluidly connected to the inspiratory circuit 60 of the TLV system S.

As shown in **figure 1****,** the fluid inlet 131 of the bag 13 is further configured to be connected to the gas injection system 30. In this case, it is possible to inject a gas mixture in the breathable liquid entering said inlet 131.

Advantageously, the backing device 11, the at least one plate 12 and the bag 13 of the hat exchange system 10 are configured to define a fluid duct 134. Said fluid duct 134 extends between the fluid inlet 131 and the fluid outlet 132 of the bag 13. Said fluid duct 134 will be later described in more details.

### The plate 12

The at least one plate 12 is configured to be at a controlled temperature.

Accordingly, said plate 12 is preferably made of a material with good thermal conductivity, such as for instance aluminum.

Advantageously, by maintaining the plate 12 at a controlled temperature, it is possible to heat or cool the breathable liquid in the fluid duct 134, before introducing it in the inspiratory circuit 60.

More precisely, if the controlled temperature of the plate 12 is lower than the temperature of the breathable liquid in the fluid duct 134, a heat transfer can occur from the breathable liquid in the fluid duct 134 to the plate 12. Consequently, the temperature of the breathable liquid in the fluid duct 134 is decreased.

If the controlled temperature of the plate 12 is higher than the temperature of the breathable liquid in the serpentine 134, a heat transfer can occur from the plate 12 to the breathable liquid in the fluid duct 134. Consequently, the temperature of the breathable liquid in the fluid duct 134 is increased.

The rate of temperature change depends on the difference between the temperature of the breathable liquid in the fluid duct 134 and the temperature of the plate 12. More precisely, the temperature change is most rapid when said difference is higher.

As aforementioned, the TLV system S may comprise a controller. Said controller may be configured to control the temperature of the breathable liquid in the fluid duct 134 to ensure that it is comprised within a predetermined range. Preferably, said predetermined range is between 20°C and 45°C.

**Figure 1** illustrates a TLV system S in which the breathable liquid treatment unit 1 comprises a thermal unit 20. This thermal unit 20 comprises a tank 21 configured to contain a fluid (for instance, water) at a predetermined temperature.

In this case, the pump assembly 5 of the TLV system S may further comprise one or more pumps 51 for pumping said fluid from the tank 21 of the thermal unit 20 towards the plate 12, so as to bring or maintain the plate 12 at a controlled temperature.

Preferably, the fluid is pumped from the tank 21 towards the plate 12 of the heat exchange system 10 at a predetermined flow rate.

For instance, the plate 12 may comprise at least one internal channel fluidly connected to the tank 21 of the thermal unit 20. In this case, the plate 12 has a thickness which is large enough to host the internal channel. The internal channel preferably extends non-linearly inside the plate 12, but it has a plurality of folds, so as to cover the whole surface of the plate 12. This ensures that the whole plate 12 is heated or cooled at the desired temperature homogeneously.

However, in an alternative embodiment, the plurality of folds does not cover the whole surface of the plate 12. This embodiment allows to obtain a heat exchange between the breathable liquid and the portion of the plate 12 comprising the channel.

For instance, the internal channel may have a cross section having a diameter comprised between 1 mm and 20 mm.

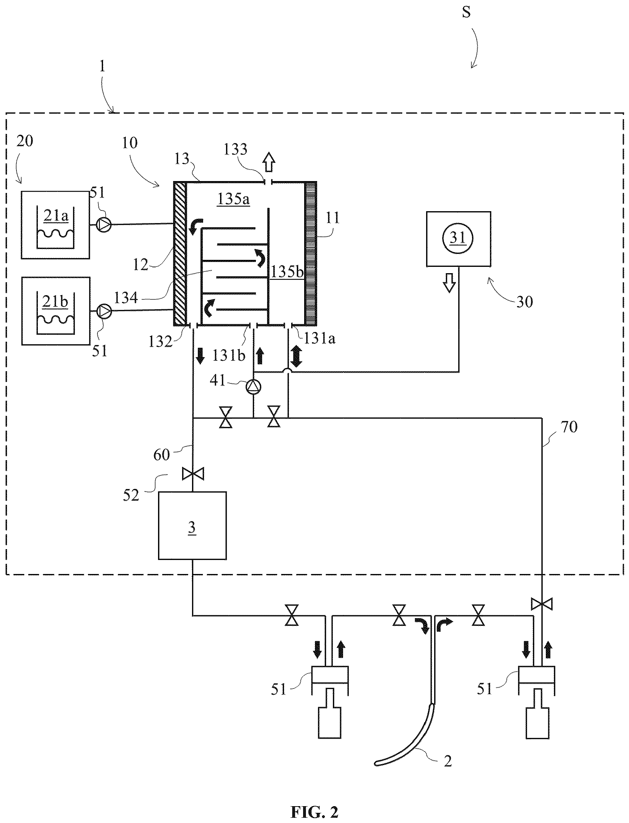

**Figure 2** illustrates another TLV system S comprising a thermal unit 20 which comprise a first tank 21a and a second tank 21b configured to contain a cold fluid and a hot fluid, respectively.

The controlled temperature of the plate 12 may be regulated for instance by modifying the flow rate of the fluids pumped towards the plate 12 and/or by modifying the temperature of the tanks 21a, 21b.

In the exemplary embodiment of **figure 2****,** the at least one plate 12 may comprise two internal channels: a first channel configured to receive the cold fluid from the first tank 21a, and a second channel configured to receive the hot fluid from the second tank 21b.

The fluid in the tank 21a, 21b may be water. In this case, the first tank 21a preferably allows to maintain the water at a temperature comprised between 0°C and 20°, more preferably between 4°C and 10°C; whereas the second tank 21b preferably allows to maintain the water at a temperature comprised between 40°C and 90°C, more preferably between 40°C and 60°C.

The plate 12 may comprise resistive elements or Peltier cells 22. For instance, said resistive elements or Peltier cells 22 may be mounted on a surface of the at least one plate 12. In this case, the heat exchange system10 may comprise a current source configured to feed the resistive elements or Peltier cells 22.

In this case, the breathable liquid treatment unit 1 may not comprise thermal unit 20.

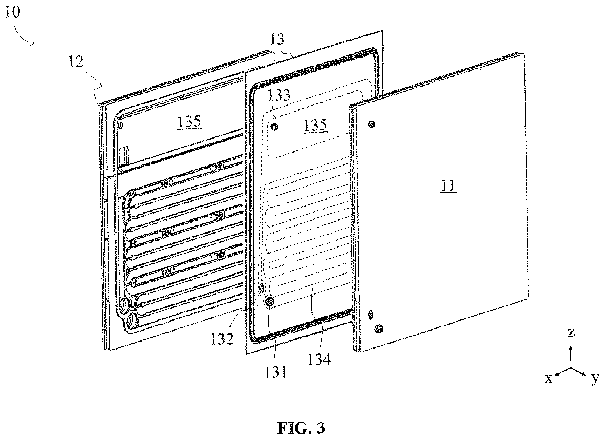

The plate 12 is better shown on **figure 3****,** which shows an exploded view of a heat exchange system 10 of the invention.

In this example, the plate 12 comprises a relief pattern. Therefore, when the bag 13 is sandwiched between the backing device 11 and the plate 12, the fluid duct 134 which is formed has the same shape as the relief pattern. More precisely, the dotted line of figure 3 represents the portion of the bag 13 which is compressed between the backing device 11 and the plate 12. The fluid duct 134 is formed inside said portion.

### The backing device 11

The backing device 11 is configured to provide mechanical stability to the heat exchange system 10.

Accordingly, said backing device 11 is preferably made of a hard material which is resistant to deformation.

For instance, said backing device 11 may be made of steel.

The backing device 11 may be a plate. For instance, it may be a plate parallel to the plate 12 configured to be at the controlled temperature. One example of this embodiment is illustrated in **figure 3****.**

In this case, the backing device 11 and the plate 12 are separated by a constant distance. Preferably, the distance between the backing device 11 and the plate 12 is comprised between 0 mm and 20 mm, more preferably between 4 mm and 15 mm.

In the example of **figure 3****,** the breathable liquid treatment unit 1 comprises a backing device 11 and a plate 12.

Alternatively, the breathable liquid treatment unit 1 may comprise more than one backing devices 11 and/or more than one plate 12 configured to be at the controlled temperature.

As shown in **figure 3****,** the backing device 11 and the plate 12 may have a surface, on a vertical plane xz, which is equal or greater than the surface of the bag 13 on said plane. Advantageously, this embodiment ensures that the whole bag 13 is sandwiched between the backing device 11 and the plate 12.

Alternatively, the backing device 11 and the plate 12 may have a surface which is smaller than the surface of the bag 13.

In this case, only a portion of the bag 13 is sandwiched between the backing device 11 and the plate 12. Advantageously, this embodiment ensures that only a fraction of the breathable liquid present inside the sandwiched portion of the bag 13 can exchange heat with the plate 12.

The backing device 11 may also be configured to be at a controlled temperature. For instance, it may be connected to the thermal unit 20. In this case, the fluid duct 134 is advantageously surrounded by components at the desired temperature (*i.e.,* the backing device 11 and the plate 12). The area available for heat exchange is thus maximized.

### The bag 13

As aforementioned, the bag 13 is sandwiched between the backing device 11 and the plate 12.

Preferably, the bag 13 is a disposable bag 13.

By "disposable" it is meant that the bag 13 is configured to be thrown away after a single use.

Advantageously, a disposable bag 13 allows to minimize the manual operations, thereby reducing the risk of contaminations.

For instance, the bag 13 may be a sterile bag, or it may be configured to be sterilized.

Preferably, the bag 13 has a volume comprised between 1 l and 7 l.

The bag 13 comprises a first inlet 131 configured to receive a breathable liquid circulating in the expiratory circuit 70.

The bag 13 comprises a fluid outlet 132 configured to evacuate the breathable liquid, once it has flowed through the fluid duct 134. The evacuated liquid, due to is passage in the fluid duct 134, has a temperature and/or gas composition different from that of the liquid received at the first inlet 131.

Advantageously, the bag 13 is configured to be inflated so that a target pressure is reached inside the fluid duct 134. Preferably, the bag 13 is configured to be inflated up to 2 bar.

During inflation, two opposite surfaces of the bag 13 tend to be spaced apart, respectively towards the plate 12 and the backing device 11.

At the target pressure, said two opposite surfaces of the bag 13 are compressed again the plate 12 and the backing device 11, respectively. This ensures the presence of an optimal thermal contact conductance between the plate 12 and the fluid duct 134 at the target pressure. Hence, heat conduction can occur by thermal contact conductance between the plate 12 and breathable liquid in the fluid duct 134, via the bag 13.

Moreover, when the target pressure is reached inside the fluid duct 134, the thermal contact resistance existing between the contacting surfaces of the bag 13 and of the plate 12 is reduced.

As shown in **figure 3****,** the bag 13 may comprise two sheets joined together on their edges. In this case, when the bag 13 is inflated by the gas injection system 30, a first sheet is moved towards the at least one plate 12, and a second sheet is moved towards the backing device 11.

The bag 13 may be configured to be maintained at a temperature comprised between 10°C and 50°C.

The bag 13 has a small thickness, preferably smaller than 1 mm; more preferably, smaller than 0.5 mm.

Preferably, the bag 13 is made of a material having a thermal resistance comprised between 0.05 and 0.5 W/mK, advantageously between 0.1 and 0.2 W/mK.

Preferably, the bag 13 may be made of a plastic material. Advantageously, it may be made of polyurethane, for instance thermoplastic polyurethane (TPU). Polyurethane is resistant to heat and pressure. Moreover, it has a low thermal resistance.

Alternatively, the bag 13 may be made of ethylene vinyl acetate (EVA), or polyvinyl chloride (PVC).

A bag 13 made of a material with low thermal resistance, and/or having a small thickness allows to reduce the thermal contact resistance between the bag 13 and the plate 12. Indeed, the thermal contact resistance between contacting surfaces is also affected by the material and the thickness of the contacting surfaces.

As shown in **figure 3****,** the bag 13 further comprises a gas outlet 133 configured to expel a gas from the fluid duct 134. Preferably, the gas outlet 133 is configured to expel a gas from a reservoir 135 communicating with the fluid duct 134.

Advantageously, the gas outlet 133 allows to remove CO₂ from the breathable liquid in the fluid duct 134, so that the breathable liquid introduced in the inspiratory circuit 60 via the first outlet 132 of the bag 13 is a CO₂-deprived two-phase mixture.

A fraction of the CO₂ present in the fluid duct may be evacuated from the gas outlet 133.

Alternatively, all the CO₂ present in the fluid duct is evacuated via the gas outlet 133.

A capnometer may be installed at the gas outlet 133, so as to measure the concentration of CO₂ in the evacuated gas.

Moreover, a pressure control valve may be mounted on the gas outlet 133 of the bag 13. The pressure control valve allows to increase or decrease the amount of gas which is expelled from the gas outlet 133.

In addition to CO₂, some other gas may be evacuated via the gas outlet 133. For instance, some O₂ may be expelled from the gas outlet 133.

The bag 13 may have more than one fluid inlet 131 and/or more than one fluid outlet 132.

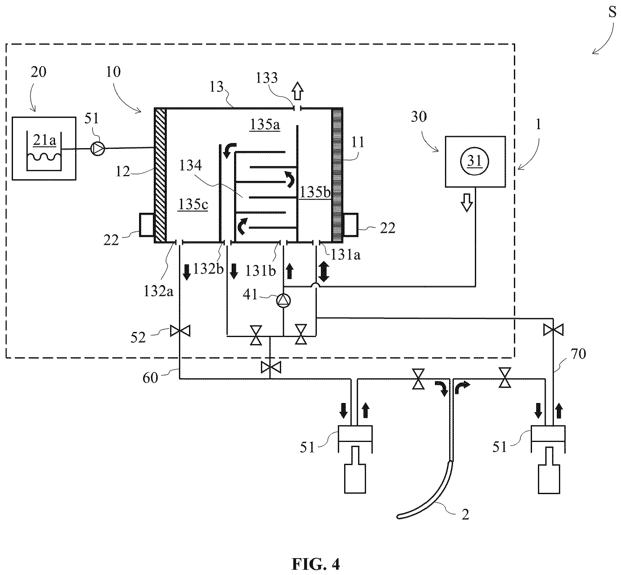

**Figure 4** shows a TLS system S in which the bag 13 has one fluid port 131a, one fluid inlet 131b and two fluids outlet 132a, 132b.

The fluid port 131a is advantageously configured to allow a bidirectional flow of the breathable liquid.

In this example, the bag 13 comprises:
- a fluid port 131a configured to receive or to deliver a breathable liquid circulating in the expiratory circuit 70;
- a fluid inlet 131b configured to receive a breathable liquid pumped by means of the pump 41 of the pumping system 40, and a gas mixture from the gas injection system 30;
- a first fluid outlet 132a configured to deliver the breathable liquid, once it has flowed through the fluid duct 134, into the inspiratory circuit 70;
- a second fluid outlet 132b configured to deliver the breathable liquid, once it has flowed through the fluid duct 134, to the pump 41 of the pumping system 40;
- a gas outlet 133 configured to expel a gas from the fluid duct 134.

### The gas injection system 30

As aforementioned, the breathable liquid treatment unit 1 also comprises a gas injection system 30.

The gas injection system 30 is configured to be connected to an inlet of the bag 13.

The gas injection system 30 is configured to inflate the bag 13. More precisely, the gas injection system 30 is configured to inject the breathable liquid comprising the gas mixture in the fluid inlet 131 of the bag 13, so as to inflate said bag 13 until a target pressure is reached inside the fluid duct 134.

The gas injection system 30 provide a twofold advantage: (i) it allows to reach, inside the fluid duct 134, the target pressure which optimizes the heat exchange between the breathable liquid and the plate 12, and (ii) it allows to introduce the gas mixture in the breathable liquid which is in the fluid duct 134.

The injection of the gas mixture into the breathable liquid allows to obtain a two-phase mixture comprising the injected gas mixture and the breathable liquid.

Therefore, the gas injection system 30 allows to obtain heat transfer and gas exchange to the breathable liquid in the fluid duct 134, simultaneously.

For instance, the gas injection system 30 may be configured to inject air, preferably medical air.

Alternatively, the gas injection system 30 may be configured to inject a mixture of air and O₂.

As aforementioned, the bag 13 comprises a gas outlet 133 configured to expel a gas separated from the breathable liquid. In some cases, some O₂ may also be expelled from said gas outlet 133.

More precisely, the amount of O₂ expelled from the gas outlet 133 depends on the composition of the gas injected in the fluid duct 134 by the gas injection system 30. The higher is the concentration of O₂ in the injected gas, the higher the amount of O₂ expelled.

Preferably, the gas injection system 30 is configured to inject a gas mixture comprising O₂ in a concentration between 21% and 100%.

The concentration of O₂ in the breathable liquid can be controlled on the basis of the concentration of O₂ in the injected gas.

The gas injection system 30 may comprise a mixing chamber 31 which comprises a nozzle configured to generate gas micro-bubbles.

For instance, the nozzle may be a Venturi-type nozzle.

By "micro-bubbles" it is meant gas bubbles having a diameter smaller than 10 mm. For instance, said micro-bubble size may be obtained via a turbulent flow regimen.

This micro-bubble size ensures that the bubbles are uniformly distributed in the two-phase mixture, thereby optimizing the heat exchange. Moreover, bubbles of this size have an equivalent surface which maximizes the gas exchange.

### Pumping system 40

The breathable liquid treatment unit 1 according to the invention further comprises a pumping system 40.

The pumping system 40 comprises a pump 41 configured to inject the breathable liquid in the fluid duct 134 via the fluid inlet 131. For instance, said pump 41 may be a centrifugal pump.

More precisely, the breathable liquid treatment unit 1 comprises a recirculating circuit configured to connect the fluid outlet 132 and the fluid inlet 131 so as to define a closed circuit with the fluid duct 134, and the circulation of the breathable liquid inside said recirculating circuit is ensured by the pump 41 of the pumping system 40.

Therefore, the circulation of the breathable liquid inside the recirculating circuit comprises:
- circulating inside the fluid duct 134;
- being withdrawn from the fluid outlet 132 after having circulated in the fluid duct 134;
- circulating inside the recirculating circuit;
- being reinjected inside the fluid duct 134 via the fluid inlet 131.

By reinjecting, by means of the pump 41 of the pumping system 40, the treated breathable liquid inside the fluid duct 134 via the fluid inlet 131, it is possible to provide several successive treatments (for instance: carbon dioxide removal, oxygen addition, heat exchange or a combination thereof) to the breathable liquid, before introducing it in the inspiratory circuit 60.

The number of treatments per minute depends on the flow rate at which the breathable liquid is pumped by the pump 41.

As shown in **figure 4****,** each of: the inspiratory circuit 60, the expiratory circuit 70, and the recirculating circuit comprise at least one valve 52 (referenced only once in each figure for clarity sake). The valve opening and closure allow to force or to block the circulation of the breathable liquid inside the recirculating circuit.

Of note, in this example, the pump 41 is located upstream of the gas exchange system 30 in the recirculating circuit. However, in an alternative embodiment, the pump 41 is located downstream of the gas exchange system, or it is combined with said gas exchange system 30.

Preferably, the breathable liquid is pumped by the pump 41 of the pumping system 40 from the fluid outlet 132 to the fluid inlet 131 at a predetermined flow rate.

As aforementioned, the bag 13 may have more than one fluid ports, inlets and/or outlets 131a, 131b 132a, 132b. In this case, the pump 41 is configured to pump the breathable liquid from at least one fluid outlet 132a, 132b to a fluid inlet 131, 131b.

One example of this embodiment is show in **figure 4****.** In this case, the recirculating circuit puts in communication the fluid outlet 132b with the fluid inlet 131b.

The direction of circulation of the breathable liquid inside the recirculating circuit also depends on the opening and closure of the valves 52.

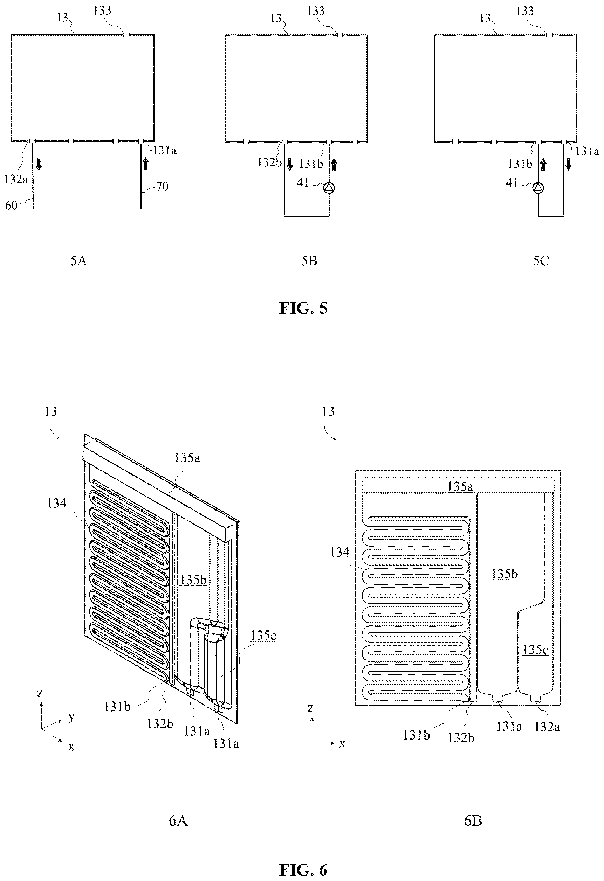

**Figure 5** is a diagram illustrating schematically the recirculating circuit in more details.

More precisely, in **figure 5A** the valves block the circulation of the breathable liquid in the recirculation circuit. Therefore, the breathable liquid circulates in the expiratory circuit 70, then in the fluid duct 134, then in the inspiratory circuit 60. **Figures 5B-5C** shows the circulation of the breathable liquid in the recirculation circuit in a first direction and in a second direction, respectively.

In **figures 5A-5C** the valves 52, and the fluid duct 134 inside the bag 13 are not illustrated for intelligibility purposes.

### The fluid duct 134

As aforementioned, at the target pressure, the fluid duct 134 is compressed between the plate 12 and the backing device 11. Therefore, a large surface of the fluid duct 134 is in contact with the plate 12 at the controlled temperature. This large contact surface allows to achieve a heat transfer which is much faster when compared to tubular heat exchange system, in which only a limited portion of the fluid duct is in contact with cold or hot pipes.

The fluid duct 134 extends between the fluid inlet 131 and the fluid outlet 132 of the bag 13, and it has a length L which is longer than the distance between said fluid inlet and outlet 131, 132.

Advantageously, the length L of the fluid duct 134 is at least twice the distance between the fluid inlet 131 and the fluid outlet 132.

For instance, the bag 13 may comprise two sheets joined together on their edges. In this case, the length L of the fluid duct 134 is preferably more than twice the longest edge of the sheets.

For instance, the fluid duct 134 may comprise a serpentine-shaped portion, a spiral-shaped portion, or a combination thereof. These embodiments allow to obtain a fluid duct 134 inside the bag 13 which is longer more than twice the distance between the fluid inlet 131 and the fluid outlet 132.

Advantageously, a long fluid duct 134 allows to increase the residence time of the breathable liquid in the heat exchange system 10, and to achieve a better separation of the gas.

Preferably, the volume of the fluid duct 134 is comprised between 0.81 and 1.5 l.

### The reservoir 135

As aforementioned, the fluid duct 134 may advantageously comprise a reservoir 135, 135a fluidly connected to the gas outlet 133.

For instance, the fluid reservoir 135 has a volume comprised between 0.1 1 and 1 l.

The fluid reservoir 135, 135a is configured to separate a gas from the breathable liquid. More precisely, the reservoir 135, 135a ensures the separation of a gas from the breathable liquid and it also allows to contain the gas after separation (i.e., the gas removed from the breathable liquid). For instance, the reservoir may be located at the top of the serpentine in a vertical direction, *i.e.,* a direction parallel to the local gravity direction, so as to take advantage of the gravity to separate the gas from the breathable liquid.

As aforementioned, a pressure control valve may be mounted on the gas outlet 133 of the bag 13. The pressure control valve is configured to regulate the pressure in the reservoir 135. By modifying the pressure in the reservoir 135, it is possible to increase or decrease the amount of gas which is expelled from the gas outlet 133.

Preferably, the pressure control valve is configured to regulate the pressure in the reservoir 135 so that it is in a range comprised between 0.1 bar and 0.4 bar.

### The second, or expiratory, reservoir 135b

The TLS system S may further comprise a second reservoir 135b (also called expiratory reservoir or expiratory buffer bag) fluidly connected to the fluid port 131a of the bag 13. This embodiment advantageously allows to obtain a constant flow rate of the breathable liquid inside the fluid duct 134 and in the gas exchange system, thereby improving the heat and gas exchange occurring in said fluid duct.

The constant flow rate may be comprised between 10 l/min and 15 l/min. In absence of the second reservoir 135b, the flow rate would not be constant. More precisely, the flow rate would be lower when the expiratory pump 51 is off, and it would be greater when the expiratory pump 51 is on.

**Figure 6** shows a second reservoir 135b integrated in the bag 13. Alternatively, said second reservoir 135b may be external to the bag 13.

Advantageously, the second reservoir 135b also prevents an overload of the expiratory pump 51.

More precisely, the fluid port 131a allows the breathable liquid to be introduced inside the second reservoir before reaching the fluid duct. The collection of the liquid inside the second reservoir, before introduction in the fluid duct, prevents an excessive power consumption by the expiratory pump.

The second reservoir 135b may be configured to be filled with the breathable liquid, which has flown in the fluid duct 134, by overflow.

Preferably, the second reservoir 135b has a volume greater than 1 l and up to 4,5 l. For instance, the second reservoir 135b may have a volume comprised between 31 and 3,5 1.

These volumes ensure that enough breathable liquid can be contained inside the second reservoir 135b.

### The third, or inspiratory, reservoir 135c

The fluid duct 134 may further comprise a third reservoir 135c (also called inspiratory reservoir or inspiratory buffer bag) fluidly connected to the fluid outlet 132a of the bag 13. Said third reservoir 135c may be filled with the breathable liquid which has flown in the fluid duct 134 by overflow.

The presence of the third reservoir 135c advantageously allows to obtain a TLV system S of compact dimensions. Indeed, in this case TLV system S does not require the presence of buffer bags 3. Therefore, the volume of breathable liquid circulating liquid inside the TLV system S is reduced, and the inspiratory circuit 60 is shorter.

Moreover, the third reservoir 135c further allows to minimize the thermal losses because the breathable liquid which has been treated in the breathable liquid treatment unit 1 can reach more rapidly the inspiratory pump 51.

In the example of **figure 6****,** the third reservoir 135c is integrated in the bag 13. Alternatively, said third reservoir 135c may be external to the bag 13.

A bag 13 having more than one of the reservoirs 135, 135a, 135b, 135c described hereabove allows to obtain a "all-in-one" bag 13.

For instance, the "all-in-one" bag 13 of **figure 6** comprises a reservoir 135a for separating a gas from the breathable liquid, a second reservoir 135b and a third reservoirs 135c.

Advantageously, an "all-in-one" bag 13 ensures that the breathable liquid is maintained at the same pressure during the different phases of the breathable liquid treatment.

Preferably, in the "all-in-one" bag 13 of **figure 6****,** the second and third reservoirs 135b, 135c do not exchange heat with the plate 12. For instance, the plate 12 may be smaller than the bag, so that only the portion of the bag 13 comprising the fluid duct 134 is sandwiched between the plate 12 and the backing device 11.

### The patterns

As aforementioned, the plate 12 may comprise a relief patter. Said pattern is on a surface of the plate 12 which faces the bag 13.

Alternatively, the pattern on the plate 12 may be a recess pattern.

In this case, the fluid duct 134 is defined by the portion of the bag 13 which is sandwiched between the backing device 11 and the relief pattern on the plate 12. In this case, when the two-phase mixture is injected in the bag 13, two opposite surfaces of the bag 13 are spaced apart where the relief pattern is not present.

The thickness of the relief pattern, measured along a direction which is normal to the plate 12, is preferably comprised between 5 mm and 15 mm. In this case, when the two-phase mixture is injected in the bag 13, two opposite surfaces of the bag 13 are spaced apart where recesses of the pattern are present. Inversely, said surfaces cannot be spaced apart in the non-recessed portion of the plate 12.

The depth of the recesses, measured along a direction which is normal to the plate 12, is preferably comprised between 5 mm and 15 mm.

In combination, or in alternative, to the pattern on the plate 12, the backing device 11 and/or on the bag 13 may comprise a pattern.

For instance, the backing device 11 may comprise a pattern complimentary to the pattern on the at least one plate 12.

One example of a pattern on the bag 13 is illustrated in **figure 5****.** In this example, the bag 13 has two surfaces joined together on their edges. Said surfaces are further joined together along a sealed boundary (for instance, heat sealed) comprised inside their edges. In this case, the pattern is defined by the sealed boundary.

The pattern on the bag 13 is configured to define an inflatable compartment within the bag 13, corresponding to the non-sealed portion of the bag 13 and delimited by the sealed boundary.

Said inflatable compartment is configured to be inflated by the gas injection system 30. The remaining portion of the bag 13, *i.e.,* the portion external to the sealed boundary, is not inflated.

Advantageously, having the pattern on the bag 13 allows to improve the tightness of the fluid duct 134, thanks to the sealed boundary. Therefore, this embodiment prevents the leaking of the breathable liquid out of the fluid duct 134.

For instance, **figure 6** shows a bag 13 comprising a serpentine pattern and three reservoir patterns, so as to define a fluid duct 134 comprising a serpentine duct 134 and three reservoirs 135a, 135b, 135c.

Alternatively, the reservoirs 135a, 135b, 135c may be external to the bag 13.

The bag 13 of **figure 6****,** may be located between a backing device 11 and a plate 12 which do not comprise any patter. Alternatively, the backing device 11 and/or the plate 12 may comprise a pattern complimentary to the pattern on the bag 13 so as to increase the contact surface.

### The method

Operation of the TLV system S will now be disclosed.

During an initial step, the plate 12 is brought at the desired controlled temperature.

During step S10, the gas injection system 30 injects a gas mixture comprising oxygen into the breathable liquid, so as to obtain a two-phase mixture comprising the gas mixture and the breathable liquid.

Then, during a step S20, the gas injection system 30 injects the gas mixture through the fluid inlet 131 of the bag 13. The injection step S30 allows to inflate the bag 13.

During step S30, a pump 51 of the pump assembly 5 pumps a breathable liquid in the fluid duct 134 through the fluid inlet 131 of the bag 13.

Steps S20 and S30 may be performed simultaneously.

The step S10 of introducing a gas mixture comprising oxygen into the breathable liquid may comprise:
- introducing a gas mixture comprising oxygen into a mixing chamber 31 with a nozzle;
- introducing the breathable liquid into the mixing chamber 31;
- generating a two-phase mixture comprising the breathable liquid and gas micro-bubbles by conducting the gas mixture and the breathable liquid through the nozzle of the mixing chamber 31;
- introducing the two-phase mixture in the fluid duct 134 through the fluid inlet 131 of the bag 13.

Then during a step S40, the two-phase mixture is conducted from the fluid inlet 131 to the fluid outlet 132 of the bag 13.

Advantageously, during said step S40, a gas may also be evacuated from the gas outlet 133 of the bag 13. In this case, step S40 further comprises:
- evacuating O₂ and CO₂ from the breathable liquid via the second outlet 133 of the bag 13;
- collecting a CO₂-deprived two-phase mixture at the fluid outlet 132 of the bag 13.

By "CO₂ deprived" it is meant that the CO₂ concentration in the two-phase mixture collected at the fluid outlet 132 is less than half of the initial CO₂ concentration, *i.e.* than the CO₂ concentration in the breathable liquid.

The gas evacuation step allows to obtain a simultaneous oxygenation of an expired breathing liquid, via the injection of oxygen in step S30, and CO₂ removal via the gas outlet 133. Thus, a CO₂-deprived breathable liquid can be collected at the fluid outlet 132, and introduced in the inspiratory circuit 60 of the TLV system S.

Preferably, the gas mixture is injected in the fluid inlet 131 of the bag 13 at a flow rate comprised between 0 L/min and 20 L/min, more preferably between 10 l/min and 15 l/min.

This flow rate advantageously allows to obtain small O₂ time constant and CO₂ time constant. By O₂ and CO₂ time constant it is meant the amount of time needed to increase the O₂ concentration by 67% and decrease the CO₂ concentration by 67% in the breathable liquid.

For instance, when the gas injected via the gas injection system is 100% oxygen, it is possible to obtain O₂ time constant smaller than 15 seconds, and CO₂ time constant smaller than 30 seconds.

Then, during a step S50, the breathable liquid is pumped by the pump 41 of the pumping system 40 from the fluid outlet 132 to the fluid inlet 131 of the bag 13. This step allows to circulate the breathable liquid inside a closed circuit comprising the fluid duct 134 and the recirculating circuit. Therefore, the breathable liquid can circulate a predetermined number of times inside said closed circuit, before being introduced in the inspiratory circuit 60 in a step S60.

During step S60, the treated breathable liquid is introduced in the inspiratory circuit 60 of the TLV system S. Therefore, said treated breathable liquid can be injected into the endotracheal tube 2 by means of the inspiratory pump 51.

During a monitoring step, the temperature of the plate 12 is monitored. Preferably, the temperature of the plate 12 is continuously monitored.

During a control step, the temperature of the plate 12 is modified. In this step, the temperature of the plate 12 is modified depending on the desired temperature of the breathable liquid. The temperature of the plate 12 is further modified based on the desired rate of temperature change of said breathable liquid.

For instance, in order to obtain a rapid decrease of the temperature of the breathable liquid in the fluid duct 134, the temperature of the plate 12 can be controlled to be as low as 0°C. In order to obtain a rapid increase of the temperature of the breathable liquid in the fluid duct 134, the temperature of the plate 12 can be controlled to be as high as 60°C. In order to maintain the temperature of the breathable liquid in the fluid duct 134 constant, the temperature of the plate 12 is controlled so as to be equal to the temperature of the breathable liquid in the fluid duct 134.

While various embodiments have been described and illustrated, the detailed description is not to be construed as being limited hereto. Various modifications can be made to the embodiments by those skilled in the art without departing from the scope of the protection as defined by the claims.

## Claims

1. A total liquid ventilation system (S) configured to deliver a breathable liquid to the lungs of a mammal, the total liquid ventilation system comprising an inspiratory circuit (60), an expiratory circuit (70), and a breathable liquid treatment unit (1), the breathable liquid treatment unit (1) comprising:
- a gas injection system (30) configured to inject a gas mixture into the breathable liquid, so as to obtain a two-phase mixture;
- a heat exchange system (10) configured to heat and/or cool the two-phase mixture, the heat exchange system (10) comprising:
• a backing device (11) configured to provide mechanical support;
• at least one plate (12) configured to be at a controlled temperature; and
• a bag (13) located in between the at least one plate (12) and the backing device (11), the bag (13) comprising at least two opposite surfaces, a fluid inlet (131) fluidly connected to the expiratory circuit (70), and a fluid outlet (132) fluidly connected to the inspiratory circuit (60);
- a pumping system (40) comprising a pump (41) for circulating the two-phase mixture from the fluid inlet (131) to the fluid outlet (132) through the fluid duct (134); and
the backing device (11), the at least one plate (12), and the bag (13) being configured to define a fluid duct (134) extending between the fluid inlet (131) and the fluid outlet (132) of the bag (13), the fluid duct (134) having a length which is larger than the distance between the fluid inlet (131) and the fluid outlet (132).

2. The total liquid ventilation system (S) according to claim **1,** further comprising:
- a recirculating circuit configured to connect the fluid outlet (132) and the fluid inlet (131) so as to define a closed circuit with the fluid duct (134); and
- wherein the pump (41) is for circulating the breathable liquid in the recirculating circuit.

3. The total liquid ventilation system (S) according to claim **1** or claim **2,** wherein the bag (13) further comprises a gas outlet (133) and a pressure control valve mounted on said gas outlet (133).

4. The total liquid ventilation system (S) according to any one of claims **1** to **3,** wherein the bag (13) comprises a sealed boundary, and wherein the fluid duct (134) is defined by the non-sealed portion of the bag (13) surrounded by said sealed boundary.

5. The total liquid ventilation system (S) according to any one of claims **1** to **4,** wherein the at least one plate (12) and/or the backing device (11) comprises a relief pattern or a recess pattern.

6. The total liquid ventilation system (S) according to any of claims **1** to **5,** wherein the backing device (11) is a plate parallel to the at least one plate (12), and separated from said at least one plate (12) by a distance comprised between 0 mm and 20 mm, preferably between 4 mm and 15 mm.

7. The total liquid ventilation system (S) according to any of claims **1** to **6,** wherein the fluid duct (134) comprises a serpentine duct extending between the fluid inlet (131) and the fluid outlet (132) and a fluid reservoir (135, 135a) for separating a gas from the breathable liquid, said reservoir (135, 135a) being fluidly connected with the serpentine duct and with the gas outlet (133) of the bag (13).

8. The total liquid ventilation system according to claim **7,** wherein the fluid reservoir (135) has a volume comprised between 0.11 and 11.

9. The total liquid ventilation system (S) according to any of claims **1** to **8,** further comprising:
- a second reservoir (135b) for buffering a breathable liquid from an expiratory pump (51), and/or
- a third reservoir (135c) for buffering a breathable liquid to be delivered to an inspiratory pump (51).

10. The total liquid ventilation system (S) according to claim **9,** wherein the second reservoir (135b) and/or the third reservoir (135c) is integrated inside the bag (13).

11. The total liquid ventilation system according to any of claims **1** to **10,** wherein the gas injection system (30) comprises a gas mixing chamber (31) fluidly connected to the fluid inlet (131) of the bag (13).

12. The total liquid ventilation system any one of claims **1** to **11,** wherein the bag (13) is made of a plastic material selected from a group comprising: Thermoplastic polyurethane (TPU), Ethylene vinyl acetate (EVA), Polyvinyl Chloride (PVC).

13. A method for simultaneously providing thermal and gas exchange to a breathable liquid in a total liquid ventilation system (S) according to any one of claims **1** to **12,** the method comprising:
- injecting (S10), by means of the gas injection system (30), a gas mixture into the breathable liquid, so as to obtain a two-phase mixture;
- pumping (S30), by means of the pump (41), the two-phase mixture in the fluid duct (134) through the inlet (131) of the bag (13).

14. The method according to claim **13,** wherein the step (S10) of introducing a gas mixture into the breathing liquid comprises:
- introducing a gas mixture comprising oxygen into a mixing chamber (31) comprising a nozzle;
- introducing the breathable liquid into the mixing chamber (31);
- generating a two-phase mixture comprising the breathable liquid and gas micro-bubbles by conducting the gas mixture and the breathable liquid through the nozzle of the mixing chamber (31).

15. The method according to claim **13** or claim **14,** further comprising:
- conducting (S40) the two-phase mixture from the fluid inlet (131) to the fluid outlet (132) of the bag (13);
- pumping (S50), by means of the pump (41), the two-phase mixture from the fluid outlet (132) to the fluid inlet (131).

## Patentansprüche

1. System (S) zur vollständigen Flüssigkeitsbelüftung, das dazu konfiguriert ist, der Lunge eines Säugetiers eine atembare Flüssigkeit zuzuführen, wobei das System zur vollständigen Flüssigkeitsbelüftung einen Inspirationskreislauf (60), einen Exspirationskreislauf (70) und eine Einheit (1) zur Behandlung mit atembarer Flüssigkeit umfasst, wobei die Einheit (1) zur Behandlung mit atembarer Flüssigkeit Folgendes umfasst:
- ein Gasinjektionssystem (30), das dazu konfiguriert ist, eine Gasmischung in die atembare Flüssigkeit zu injizieren, um eine Zweiphasenmischung zu erhalten;
- ein Wärmeaustauschsystem (10), das dazu konfiguriert ist, die Zweiphasenmischung zu erwärmen und/oder zu kühlen, wobei das Wärmeaustauschsystem (10) Folgendes umfasst:
• eine Rückhaltevorrichtung (11), die dazu konfiguriert ist, mechanische Unterstützung bereitzustellen;
• mindestens eine Platte (12), die dazu konfiguriert ist, eine kontrollierte Temperatur aufzuweisen; und
• einen Beutel (13), der sich zwischen der mindestens einen Platte (12) und der Rückhaltevorrichtung (11) befindet, wobei der Beutel (13) mindestens zwei gegenüberliegende Oberflächen, einen Fluideinlass (131), der mit dem Exspirationskreislauf (70) fluidisch verbunden ist, und einen Fluidauslass (132), der mit dem Inspirationskreislauf (60) fluidisch verbunden ist, umfasst;
- ein Pumpsystem (40), das eine Pumpe (41) zum Zirkulieren der Zweiphasenmischung vom Fluideinlass (131) zum Fluidauslass (132) durch den Fluidkanal (134) umfasst; und
wobei die Rückhaltevorrichtung (11), die mindestens eine Platte (12) und der Beutel (13) dazu konfiguriert sind, einen Fluidkanal (134) zu definieren, der sich zwischen dem Fluideinlass (131) und dem Fluidauslass (132) des Beutels (13) erstreckt, wobei der Fluidkanal (134) eine Länge aufweist, die größer ist als der Abstand zwischen dem Fluideinlass (131) und dem Fluidauslass (132).

2. System (S) zur vollständigen Flüssigkeitsbelüftung nach Anspruch 1, das ferner Folgendes umfasst:
- einen Rezirkulationskreislauf, der dazu konfiguriert ist, den Fluidauslass (132) und den Fluideinlass (131) zu verbinden, um einen geschlossenen Kreislauf mit dem Fluidkanal (134) zu bilden; und
- wobei die Pumpe (41) zum Zirkulieren der atembaren Flüssigkeit im Rezirkulationskreislauf dient.

3. System (S) zur vollständigen Flüssigkeitsbelüftung nach Anspruch 1 oder Anspruch 2, wobei der Beutel (13) ferner einen Gasauslass (133) und ein Druckregelventil umfasst, das an dem Gasauslass (133) montiert ist.

4. System (S) zur vollständigen Flüssigkeitsbelüftung nach einem der Ansprüche 1 bis 3, wobei der Beutel (13) einen versiegelten Rand umfasst, und wobei der Fluidkanal (134) durch den nicht versiegelten Abschnitt des Beutels (13) definiert ist, der von dem versiegelten Rand umgeben ist.

5. System (S) zur vollständigen Flüssigkeitsbelüftung nach einem der Ansprüche 1 bis 4, wobei die mindestens eine Platte (12) und/oder die Rückhaltevorrichtung (11) ein Reliefmuster oder ein Vertiefungsmuster umfasst.

6. System (S) zur vollständigen Flüssigkeitsbelüftung nach einem der Ansprüche 1 bis 5, wobei die Rückhaltevorrichtung (11) eine Platte ist, die parallel zu der mindestens einen Platte (12) verläuft und von der mindestens einen Platte (12) um einen Abstand zwischen 0 mm und 20 mm, vorzugsweise zwischen 4 mm und 15 mm, getrennt ist.

7. System (S) zur vollständigen Flüssigkeitsbelüftung nach einem der Ansprüche 1 bis 6, wobei der Fluidkanal (134) einen Serpentinenkanal umfasst, der sich zwischen dem Fluideinlass (131) und dem Fluidauslass (132) erstreckt, und ein Fluidreservoir (135, 135a) zum Trennen eines Gases von der atembaren Flüssigkeit, wobei das Reservoir (135, 135a) fluidisch mit dem Serpentinenkanal und mit dem Gasauslass (133) des Beutels (13) verbunden ist.

8. System (S) zur vollständigen Flüssigkeitsbelüftung nach Anspruch 7, wobei das Fluidreservoir (135) ein Volumen zwischen 0,1 L und 1 L aufweist.

9. System (S) zur vollständigen Flüssigkeitsbelüftung nach einem der Ansprüche 1 bis 8, das ferner Folgendes umfasst:
- ein zweites Reservoir (135b) zum Puffern einer atembaren Flüssigkeit von einer Exspirationspumpe (51) und/oder
- ein drittes Reservoir (135c) zum Puffern einer atembaren Flüssigkeit, die einer Inspirationspumpe (51) zugeführt werden soll.

10. System (S) zur vollständigen Flüssigkeitsbelüftung nach Anspruch 9, wobei das zweite Reservoir (135b) und/oder das dritte Reservoir (135c) in den Beutel (13) integriert ist.

11. System (S) zur vollständigen Flüssigkeitsbelüftung nach einem der Ansprüche 1 bis 10, wobei das Gasinjektionssystem (30) eine Gasmischkammer (31) umfasst, die fluidisch mit dem Fluideinlass (131) des Beutels (13) verbunden ist.

12. System (S) zur vollständigen Flüssigkeitsbelüftung nach einem der Ansprüche 1 bis 11, wobei der Beutel (13) aus einem Kunststoffmaterial besteht, das aus einer Gruppe ausgewählt ist, die Folgendes umfasst: Thermoplastisches Polyurethan (TPU), Ethylenvinylacetat (EVA), Polyvinylchlorid (PVC).

13. Verfahren zum gleichzeitigen Bereitstellen von Wärme- und Gasaustausch mit einer atembaren Flüssigkeit in einem System (S) zur vollständigen Flüssigkeitsbelüftung nach einem der Ansprüche 1 bis 12,
wobei das Verfahren Folgendes umfasst:
- Injizieren (S 10) einer Gasmischung in die atembare Flüssigkeit mit Hilfe des Gasinjektionssystems (30), um eine Zweiphasenmischung zu erhalten;
- Pumpen (S30) der Zweiphasenmischung in dem Fluidkanal (134) durch den Einlass (131) des Beutels (13) mit Hilfe der Pumpe (41).

14. Verfahren nach Anspruch 13, wobei der Schritt (S10) des Einleitens eines Gasgemisches in die atembare Flüssigkeit Folgendes umfasst:
- Einleiten einer Gasmischung, die Sauerstoff umfasst, in eine Mischkammer (31), die eine Düse umfasst;
- Einleiten der atembaren Flüssigkeit in die Mischkammer (31);
- Erzeugen einer Zweiphasenmischung, die aus der atembaren Flüssigkeit und Gasmikrobläschen besteht, indem die Gasmischung und die atembare Flüssigkeit durch die Düse der Mischkammer (31) eingeleitet wird.

15. Verfahren nach Anspruch 13 oder Anspruch 14, das ferner Folgendes umfasst:
- Leiten (S40) der Zweiphasenmischung vom Fluideinlass (131) zum Fluidauslass (132) des Beutels (13);
- Pumpen (S50) des Zweiphasengemisches vom Fluidauslass (132) zum Fluideinlass (131) mit Hilfe der Pumpe (41).

## Revendications

1. Système de ventilation liquidienne totale (S) configuré pour délivrer un liquide respiratoire aux poumons d'un mammifère, le système de ventilation liquidienne totale comprenant un circuit inspiratoire (60), un circuit expiratoire (70), et une unité de traitement de liquide respiratoire (1), l'unité de traitement de liquide respiratoire (1) comprenant :
- un système d'injection de gaz (30) configuré pour injecter un mélange de gaz dans le liquide respiratoire, de manière à obtenir un mélange biphasé ;
- un système d'échange thermique (10) configuré pour chauffer et/ou refroidir le mélange biphasé, le système d'échange thermique (10) comprenant :
• un dispositif de support (11) configuré pour fournir un support mécanique ;
• au moins une plaque (12) configurée pour être à une température contrôlée ; et
• un sac (13) situé entre la plaque (12) et le dispositif de support (11), le sac (13) comprenant au moins deux surfaces opposées, une entrée de fluide (131) reliée de manière fluide au circuit expiratoire (70), et une sortie de fluide (132) reliée de manière fluide au circuit inspiratoire (60) ;
- un système de pompage (40) comprenant une pompe (41) pour faire circuler le mélange biphasé de l'entrée de fluide (131) à la sortie de fluide (132) à travers le conduit de fluide (134) ; et
le dispositif de support (11), l'au moins une plaque (12) et le sac (13) étant configurés pour définir un conduit de fluide (134) s'étendant entre l'entrée de fluide (131) et la sortie de fluide (132) du sac (13), le conduit de fluide (134) ayant une longueur supérieure à la distance entre l'entrée de fluide (131) et la sortie de fluide (132).

2. Le système de ventilation liquidienne totale (S) selon la revendication 1, comprenant en outre :
- un circuit de recirculation configuré pour relier la sortie de fluide (132) et l'entrée de fluide (131) de manière à définir un circuit fermé avec le conduit de fluide (134) ; et
- dans lequel la pompe (41) est destinée à faire circuler le liquide respiratoire dans le circuit de recirculation.

3. Système de ventilation liquidienne totale (S) selon la revendication **1** ou la revendication **2,** dans lequel le sac (13) comprend en outre une sortie de gaz (133) et une soupape de contrôle de la pression montée sur ladite sortie de gaz (133).

4. Le système de ventilation liquidienne totale (S) selon l'une des revendications **1** à **3,** dans lequel le sac (13) comprend un bord scellé, et dans lequel le conduit de fluide (134) est défini par la partie non scellée du sac (13) entourée par ledit bord scellé.

5. Le système de ventilation liquidienne totale (S) selon l'une quelconque des revendications **1** à **4,** dans lequel l'au moins une plaque (12) et/ou le dispositif de support (11) comporte un motif en relief ou un motif en creux.

6. Le système de ventilation liquidienne totale (S) selon l'une des revendications **1** à **5,** dans lequel le dispositif de support (11) est une plaque parallèle à l'au moins une plaque (12), et séparée de ladite au moins une plaque (12) par une distance comprise entre 0 mm et 20 mm, de préférence entre 4 mm et 15 mm.

7. Le système de ventilation liquidienne totale (S) selon l'une des revendications **1** à **6,** dans lequel le conduit de fluide (134) comprend un conduit en serpentin s'étendant entre l'entrée de fluide (131) et la sortie de fluide (132) et un réservoir de fluide (135, 135a) pour séparer un gaz du liquide respiratoire, ledit réservoir (135, 135a) étant relié de manière fluidique au conduit en serpentin et à la sortie de gaz (133) du sac (13).

8. Le système de ventilation liquidienne totale selon la revendication **7,** dans lequel le réservoir de fluide (135) a un volume compris entre 0,1 L et 1 L.

9. Le système de ventilation liquidienne totale (S) selon l'une des revendications **1** à **8,** comprenant en outre :
- un deuxième réservoir (135b) pour tamponner un liquide respiratoire provenant d'une pompe expiratoire (51), et/ou
- un troisième réservoir (135c) pour tamponner un liquide respiratoire à délivrer à une pompe inspiratoire (51).

10. Système de ventilation liquidienne totale (S) selon la revendication **9,** dans lequel le deuxième réservoir (135b) et/ou le troisième réservoir (135c) est intégré à l'intérieur du sac (13).

11. Système de ventilation liquidienne totale selon l'une des revendications **1** à **10,** dans lequel le système d'injection de gaz (30) comprend une chambre de mélange de gaz (31) reliée fluidiquement à l'entrée de fluide (131) du sac (13).

12. Système de ventilation liquidienne totale selon l'une des revendications **1** à **11,** dans lequel le sac (13) est fait d'un matériau plastique choisi dans un groupe comprenant : Polyuréthane thermoplastique (TPU), éthylène-acétate de vinyle (EVA), chlorure de polyvinyle (PVC).

13. Procédé pour assurer simultanément l'échange thermique et gazeux d'un liquide respiratoire dans un système de ventilation liquidienne totale (S) selon l'une quelconque des revendications **1** à **12,** le procédé comprenant :
- injection (S10), au moyen du système d'injection de gaz (30), d'un mélange de gaz dans le liquide respiratoire, de manière à obtenir un mélange biphasé ;
- pompage (S30), au moyen de la pompe (41), du mélange biphasé dans le conduit de fluide (134) à travers l'entrée (131) du sac (13).

14. Le procédé selon la revendication **13,** dans lequel l'étape (S10) d'injection d'un mélange de gaz dans le liquide respiratoire comprend :
- introduction d'un mélange de gaz comprenant de l'oxygène dans une chambre de mélange (31) comprenant une buse ;
- introduction du liquide respiratoire dans la chambre de mélange (31) ;
- génération d'un mélange biphasé comprenant le liquide respiratoire et des microbulles de gaz en conduisant le mélange de gaz et le liquide respiratoire à travers la buse de la chambre de mélange (31).

15. Le procédé selon la revendication **13** ou la revendication **14,** comprenant en outre :
- conduite (S40) du mélange biphasé de l'entrée de fluide (131) à la sortie de fluide (132) du sac (13) ;
- pompage (S50), au moyen de la pompe (41), du mélange biphasé de la sortie de fluide (132) à l'entrée de fluide (131).
